# DEMANDE DE BREVET EUROPEEN

(11) **EP 4 403 039 A1**
(43) Date de publication de la demande: **24.07.2024**
(21) Numéro de dépôt: 24153385.0
(22) Date de dépôt: 23.01.2024
(51) Int. Cl.: A23L 7/00, A23L 7/104, A23L 7/117, A23L 11/00, A61K 8/00, A61K 47/00, A23L 19/15, A23L 19/18

(54) **BASE DE POMME DE TERRE ET DE VÉGÉTAUX AMYLACÉS**

(30) Priorité: 23.01.2023 BE 202305035
(71) Demandeur: Meurens Natural, 4650 Herve (BE)
(72) Inventeur: MALMENDIER, Yves Paul, 4910 Theux (BE); PIROTTE, Manon Mireille, 4130 Esneux (BE)
(74) Mandataire: Calysta NV

(57) **Abrégé**

La présente invention se rapporte à une base de pomme de terre et de végétaux amylacés, à son procédé de préparation respectivement à partir de pomme de terre ou de flocons de pomme de terre, ainsi qu'à l'utilisation d'une telle base selon l'invention.

## Description

La présente invention se rapporte à une base de pomme de terre et de végétaux amylacés.

La présente invention concerne en outre un procédé de préparation d'une base de pomme de terre et de végétaux amylacés selon l'invention à partir de pomme de terre.

La présente invention concerne également un procédé de préparation d'une base de pomme de terre et de végétaux amylacés selon l'invention à partir de flocons ou farine de flocons de pomme de terre.

Plus précisément, l'invention se rapporte à l'utilisation d'une telle base de pomme de terre et de végétaux amylacés selon l'invention.

La pomme de terre comme produit alimentaire est aujourd'hui considérée comme la principale denrée alimentaire au monde, elle est notamment cultivée et consommée de manière locale dans plus de 150 pays.

Du point de vue de sa composition, la pomme de terre comprend environ 78,5% d'eau, 17,6% d'amidon, 2% de vitamines et minéraux, 1,9% de protéines et 1,8% de fibres, ce qui en fait un aliment ayant des atouts nutritionnels non négligeables comme par exemple une source d'antioxydants, riche en amidon, une teneur en fibres appréciable, la prévention de certains cancers, une source de potassium et d'oligo-éléments, une source de vitamines et particulièrement B6.

En revanche, il est également reconnu qu'il faut éviter de consommer des pommes de terre crues, à cause de leur toxicité.

En outre, la pomme de terre est également utilisée dans l'industrie pharmaceutique et cosmétique, par exemple pour la fabrication de comprimés, dans la composition d'antibiotiques, de compléments vitaminés, de vaccins, de gélifiant dans les produits cosmétiques.

On se rend donc bien compte que la pomme de terre présente bon nombre d'avantages et est un produit largement utilisé, bien que sa culture et consommation se fasse toujours de manière locale à l'échelle de la planète car la congélation des pommes de terre crues n'est pas recommandée, et la conservation pour éviter la germination n'est pas non plus aisée.

Dès lors et en considérant les volumes de pommes de terre qui sont cultivés, ce produit reste local et est généralement rapidement consommé ou transformé. Cela dans le but d'éviter le long stockage qui entraîne une hausse des composés toxiques et une détérioration du goût bien qu'il ne soit déjà pas très agréable pour une pomme de terre crue.

Malheureusement, il s'avère que les dernières crises mondiales ont également entraîné une crise dans la consommation et le stockage de pommes de terre, notamment à cause de la baisse de consommation.

La baisse de consommation et de transformation industrielle des pommes de terre a entraîné un surplus de pommes de terre destinées à l'alimentation humaine qui peinent à être correctement stockées.

Diverses solutions ont alors été développées pour écouler ces stocks qui perdent en qualité au cours du temps et permettre le renouvellement des hangars avec des produits frais de meilleure qualité, comme par exemple le compostage, le stockage sur champ avant épandage ou encore l'épandage sur champ.

Par ailleurs, on connaît également le procédé transformation de pommes de terre en flocons de pomme de terre, via une cuisson, déshydratation puis broyage sous forme de flocons. En revanche, bien que les pommes de terre sous forme de flocons présentent une conservation bien améliorée, l'utilisation de tels flocons doit justifier les coûts de production. En effet, déshydrater un produit comprenant environ 75% d'eau est coûteux, la valorisation des flocons de pomme de terre doit dès lors se faire via des produits à valeur ajoutée importante.

Malheureusement, bien que ces solutions puissent présenter certains avantages, la valorisation des pommes de terre gagnerait à être améliorée.

Il existe donc un réel besoin de fournir un produit permettant la valorisation des pommes de terre alors que ces dernières étant composées à plus de 75% d'eau, soit moins de 25% de matière sèche, leur congélation n'est pas souhaitable, leur conservation n'est pas non plus aisée et doit se faire dans des conditions optimales (obscurité, ventilation, hygrométrie, température contrôlées) pour éviter la respiration, transpiration, pertes de poids, flétrissement des pommes de terre mais également éviter le développement de germes, fermentations, attaques bactériennes ou fongiques qui sont des facteurs de risques augmentant avec le temps de stockage, et ce tout en conservant un goût de pomme de terre qui soit acceptable pour le consommateur alors que le goût de la pomme de terre crue est amer et non désiré, et qui soit uniforme dans l'utilisation de la matière de départ (pomme de terre crue ou flocons de pommes de terre).

La présente invention a pour but de pallier les inconvénients de l'état de la technique en procurant une base de pomme de terre et de végétaux amylacés, sous forme liquide ou sous forme de poudre, comprenant du glucose, du sucrose et du maltose, présentant un degré Brix compris entre 65 et 85 sous forme liquide ou une teneur en matière sèche comprise entre 90% et 100% sous forme de poudre, et présentant une valeur de Dextrose Equivalent (DE) comprise entre 10 et 95.

En effet, il est apparu de manière particulièrement avantageuse qu'il était possible de fournir une base, à savoir un extrait, sous forme liquide ou sous forme solide, de poudre, étant obtenue à partir d'un mélange de pomme de terre et de végétaux amylacés, ce qui permet de valoriser les filières agricoles locales, autant pour les pommes de terre que pour les végétaux, permettant d'éviter que ces denrées ne soit jetées ou détruites sans avoir été préalablement valorisées.

En outre, les problématiques liées au goût et à la consistance de la pomme de terre sont résolues grâce à l'apport des végétaux amylacés. En effet, les végétaux amylacés, et plus particulièrement graines de ces végétaux, à savoir les céréales ou pseudo-céréales, permettent d'apporter la matière sèche manquante à la pomme de terre et de compenser le mauvais goût de la pomme de terre par un goût plus atténué mélangé à celui de la graine utilisée, par exemple une céréale.

La base, l'extrait de pomme de terre et de végétaux, de graines selon la présente invention, sous forme liquide ou solide sous forme de poudre, comprenant du glucose, sucrose et maltose, permet de fournir une alternative sucrante et fonctionnelle aux autres extraits de céréales dans l'agroalimentaire pour la préparation de nombreux produits, en la diluant et/ou en l'associant à d'autres ingrédients (lipides, vitamines, etc...). Ainsi, la base selon la présente invention permet de fournir une base prête à être utilisée directement et/ou utilisée pour la préparation de produits intermédiaires ou finaux, ayant un goût de pomme de terre léger et acceptable pour le consommateur atténué par le goût de la graine utilisée, un pouvoir sucrant, une couleur naturelle, de la viscosité et/ou de la structure.

Selon l'invention et comme bien connu dans le domaine de l'agroalimentaire, la « valeur de DE » ou valeur de dextrose équivalent mesure le degré d'hydrolyse de l'amidon contenu dans la céréale et est ainsi une mesure de la quantité de sucres réducteurs présents dans un produit sucré, exprimé en pourcentage sur une base sèche par rapport au dextrose. Ainsi, plus le DE est élevé, plus l'hydrolyse de l'amidon est poussée et plus la proportion en sucres simples (à chaîne courte) est élevée. Une hydrolyse totale de l'amidon en glucose (dextrose) correspond à un DE de 100 tandis que l'amidon lui-même aura un DE quasiment égal à zéro.

Selon l'invention, la base sous forme liquide présente un degré Brix compris entre 65 et 85. Ces valeurs de degrés Brix entrainent que la base peut être qualifiée de « sirop » ou de « concentré », notamment destinée à la préparation de boissons par dilution.

Ainsi, la base sous forme liquide selon l'invention présentant un degré Brix compris entre 65 et 85 permet à la base d'être qualifiée comme un sirop liquide ou, la base sous forme solide, sous forme de poudre selon l'invention présentant une teneur en matière sèche comprise entre 90% et 100%, pouvant être utilisé pour son pouvoir sucrant et/ou dans un produit intermédiaire ou final qui soit alimentaire, cosmétique et/ ou pharmaceutique, et la base selon l'invention présentant une valeur de Dextrose Equivalent (DE) comprise entre 10 et 95 permet de fournir un bon compromis entre la quantité en sucres, la viscosité de la base et la durée de conservation. La base de pomme de terre et de végétaux amylacés (l'extrait de pomme de terre et de végétaux) selon l'invention, sous forme liquide et/ou solide, permet ainsi de manière particulièrement avantageuse d'apporter une solution à la valorisation des pommes de terres crues ou sous forme de flocons, de promouvoir la valorisation du secteur agricole local autant pour la pomme de terre que pour les graines, et de fournir un produit facilement utilisable comme produit principal ou dans la préparation de produits intermédiaires ou finaux, ayant un goût, une viscosité, une structure, et un pouvoir sucrant acceptables et recherchés.

De préférence, la base de pomme de terre et de végétaux amylacés selon l'invention présente un degré Brix compris entre 66 et 84, de préférence entre 67 et 83, préférentiellement entre 68 et 82, de manière préférée entre 69 et 81, plus précisément entre 70 et 80, par exemple entre 71 et 80.

Avantageusement, la base de pomme de terre et de végétaux amylacés selon l'invention, présente sous forme de poudre, une teneur en matière sèche comprise entre 92% et 100%, de préférence comprise entre 92% et 98%, préférentiellement comprise entre 94% et 98%.

Préférentiellement, la base de pomme de terre et de végétaux amylacés selon l'invention présentant une valeur de Dextrose Equivalent (DE) comprise entre 15 et 90, de préférence entre 15 et 85, préférentiellement entre 15 et 89, de manière avantageusement entre 20 et 75, de préférence comprise entre 25 et 70, avantageusement comprise entre 30 et 65, plus précisément comprise entre 35 et 65, de manière avantageuse comprise entre 35 et 62.

De manière avantageuse, la base de pomme de terre et de végétaux amylacés selon l'invention présente sous forme liquide, une viscosité mesurée à 25°C comprise entre 1 et 50000 mPa.s, de préférence comprise entre 500 et 39000 mPa.s, avantageusement comprise entre 1000 et 35000 mPa.s, préférentiellement comprise entre 2000 et 30000 mPa.s, plus préférentiellement comprise entre 3000 et 28000 mPa.s, encore plus avantageusement comprise entre 4000 et 26000 mPa.s, avantageusement comprise entre 5000 et 25000 mPa.s, de manière préférée comprise entre 5000 et 21000 mPa.s.

Cela permettant une séparation facile en fin de production et des manipulations ultérieures qui sont aisées et rapides, notamment pour les prélèvements, les dosages, vidanges des containeurs et installations mais également pour fournir une base qui sera facilement utilisée dans l'industrie agro-alimentaire, pharmaceutique et/ou cosmétique en ayant une viscosité souhaitable.

Avantageusement, la base de pomme de terre et de végétaux amylacés selon l'invention présente un pH compris entre 3,5 et 7,5.

De préférence, la base de pomme de terre et de végétaux amylacés selon l'invention comprend une quantité en sucres sur matière sèche comprise entre 2% et 86%, de préférence comprise entre 3,5% et 85%, préférentiellement comprise entre 5% et 80%, avantageusement comprise entre 10% et 80%, de manière avantageuse comprise entre 15% et 80%, de manière préférée comprise entre 20% et 80%, plus préférentiellement comprise entre 25% et 80%, avantageusement comprise entre 25% et 75%, de préférence comprise entre 30% et 75%, de manière préférée comprise entre 35% et 75%, de manière encore plus préférée comprise entre 35% et 70%.

De manière préférée, la base de pomme de terre et de végétaux amylacés selon l'invention présente une teneur en potassium comprise entre 100 et 4500 ppm.

Préférentiellement, la base de pomme de terre et de végétaux amylacés selon l'invention présente une teneur en magnésium comprise entre 30 et 1000 ppm.

Avantageusement, la base de pomme de terre et de végétaux amylacés selon l'invention présente une teneur en phosphore comprise entre 40 et 3500 ppm.

De manière préférée, la base de pomme de terre et de végétaux amylacés selon l'invention présente une teneur en calcium comprise entre 20 et 200 ppm.

Avantageusement, la base de pomme de terre et de végétaux amylacés selon l'invention présente une teneur en fer comprise entre 1 et 6 ppm.

De manière particulièrement avantageuse, la base de pomme de terre et de végétaux amylacés selon l'invention présente une teneur en gluten inférieure à 100 ppm, de préférence inférieure à 80 ppm, préférentiellement inférieure à 60 ppm, avantageusement inférieure à 50 ppm, de manière particulièrement avantageuse inférieure à 40 ppm, de manière encore plus préférée inférieure à 30 ppm, idéalement inférieure à 20 ppm. Ce qui permet de fournir un produit sans gluten. Encore plus précisément, inférieure à 10 ppm, voire inférieure à 5ppm.

De préférence, les végétaux amylacés de la base selon l'invention sont des céréales, des pseudo-céréales, des poaceaes, des amaranthaceaes, des polygonaceaes, des fabaceaes, les plantes racines, les convolvulaceaes, les euphorbiaceaes, les apiaceaes, les asteraceaes et leurs mélanges.

Préférentiellement, les végétaux amylacés de la base selon l'invention sont choisies dans le groupe comprenant le riz, l'avoine, le blé, le manioc, l'épeautre, le seigle, le maïs, l'orge malté, le teff, le sorgho, le quinoa, le sarrasin, le millet, l'orge, les pois, les pois chiche, l'amaranthe, le blé dur, le tritordeum, le tournesol, et leurs mélanges.

De préférence, les végétaux amylacés et/ou la pomme de terre du procédé selon la présente invention ont subi un prétraitement de triage, nettoyage, épluchage, découpage, broyage, séchage, toastage, classification, traitements thermiques, floconnage, fractionnement, maltage et leurs mélanges. De préférence, de leur fraction et/ou de leur co-produit et/ou de leur sous-produit.

D'autres formes de réalisation de la base de pomme de terre et de graines selon la présente invention sont indiquées dans les revendications annexées.

L'invention a aussi pour objet un procédé de préparation d'une base de pomme de terre et de végétaux amylacés selon l'invention, comprenant :
- une alimentation d'une unité de préparation en végétaux amylacés,
- une alimentation d'une cuve à hydrolyse avec de la pomme de terre, et une phase aqueuse,
- au moins une première étape d'ajout d'au moins une enzyme dans la cuve à hydrolyse pour former un premier mélange de pomme de terre dans une phase aqueuse enzymatique,
- un entraînement progressif et continu pendant une période de temps prédéterminé, dans un circuit de circulation, des végétaux amylacés de l'unité de préparation par ledit premier mélange, pour former progressivement et en continu un deuxième mélange de végétaux amylacés dans la phase aqueuse enzymatique comprenant la pomme de terre,
- une première étape d'hydrolyse progressive et continue du deuxième mélange pour former un hydrolysat de pomme de terre et de végétaux amylacés,
- une étape de séparation mécanique de l'hydrolysat pour former la base de pomme de terre et de végétaux sous forme liquide, ou
- une étape de séchage de l'hydrolysat pour former la base de pomme de terre et de végétaux solide.

Il est apparu de manière particulièrement avantageuse que le procédé de préparation d'une base selon l'invention, en réalisant un entraînement progressif et continu pendant une période de temps prédéterminé, des végétaux par le premier mélange (pomme de terre dans une phase aqueuse enzymatique) permettait d'enrichir et de concentrer progressivement ce mélange.

De plus, la première étape d'hydrolyse du procédé selon l'invention a lieu également de manière progressive et continue, ce qui va permettre de former l'hydrolysat de manière contrôlée et de contrôler l'hydrolyse enzymatique du deuxième mélange (graines/végétaux amylacés dans la phase aqueuse enzymatique comprenant la pomme de terre).

Plus particulièrement, les végétaux amylacés selon l'invention sont par exemple des graines de végétaux amylacés, par exemple une farine de végétaux amylacés et/ou une farine de graines de végétaux amylacés.

Ainsi, les végétaux amylacés selon l'invention, sous forme de graine et/ou de farine sont introduits dans une unité de préparation, débouchant dans un circuit de circulation qui est une boucle de circulation entre l'unité de préparation et la cuve à hydrolyse. Ainsi, la phase aqueuse de la cuve à hydrolyse réalise une boucle de circulation entre la cuve à hydrolyse et le circuit de circulation ce qui permet l'entraînement progressif et continu de la matière première (végétaux amylacés) provenant de l'unité de préparation.

Cela permettant de manière particulièrement avantageuse, d'une part d'enrichir progressivement et en continu le premier mélange, en évitant l'introduction brutale des végétaux amylacés, par exemple des graines et/ou de la farine, en une fois dans la cuve à hydrolyse comme cela est communément réalisé dans l'art antérieur, entraînant de laborieuses étapes de mélange, une augmentation brutale de la viscosité entraînant généralement une mauvaise circulation voire un bouchage des installations industrielles.

D'autre part, en introduisant l'au moins une enzyme et en continuant l'étape d'enrichissement, on va progressivement et en continu continuer d'enrichir la phase aqueuse enzymatique comprenant la pomme de terre avec les végétaux amylacés, en évitant les inconvénients mentionnés ci-avant.

Alternativement, l'invention concerne un procédé de préparation d'une base de pomme de terre et de végétaux amylacés selon l'invention comprenant :
- une alimentation d'une cuve à hydrolyse avec une phase aqueuse,
- une série d'alimentations d'une unité de préparation en végétaux amylacés et en flocons ou farine de flocons de pomme de terre,
- au moins une première étape d'ajout d'au moins une enzyme dans la cuve à hydrolyse,
- un entraînement progressif et continu pendant une période de temps prédéterminé, dans un circuit de circulation, des végétaux amylacés et des flocons ou farine de flocons de pomme de terre de l'unité de préparation par la phase aqueuse enzymatique,
- une étape d'hydrolyse progressive et en continue du mélange de végétaux amylacés dans la phase aqueuse enzymatique comprenant les flocons ou farine de flocons de pomme de terre,
- une étape de séparation mécanique de l'hydrolysat pour former la base de pomme de terre et de végétaux sous forme liquide, ou
- une étape de séchage de l'hydrolysat pour former la base de pomme de terre et de végétaux sous forme de poudre.

Il est entendu que la série d'alimentations d'une unité de préparation en végétaux amylacés et en flocons ou farine de flocons de pomme de terre peut être une alimentation en végétaux amylacés puis une alimentation en flocons ou farine de flocons, ou bien une alimentation en flocons ou farine de flocons puis une alimentation en végétaux amylacés, ou encore une alimentation d'un mélange de végétaux amylacés et de flocons ou farine de flocons de pomme de terre.

En outre, l'entraînement progressif et continu pendant une période de temps prédéterminé, dans un circuit de circulation par la phase aqueuse enzymatique peut donc être un entraînement des végétaux amylacés puis des flocons ou farine de flocons, ou bien un entraînement des flocons ou farine de flocons puis des végétaux amylacés, ou encore un entraînement d'un mélange de végétaux amylacés et de flocons ou farine de flocons de pomme de terre.

Enfin, et de manière particulièrement avantageuse l'hydrolyse va avoir lieu de manière concomitante de la pomme de terre et des végétaux amylacés, ce qui permet de contrôler parfaitement les propriétés sucrantes de l'hydrolysat de pomme de terre et de végétaux amylacés obtenu, et non pas réaliser deux hydrolyses séparées puis mélanger les hydrolysats obtenus.

L'hydrolyse enzymatique concomitante de la pomme de terre et des végétaux amylacés, de préférence de graines et/ou de farines de végétaux amylacés, permet de former un hydrolysat contrôlé, lequel subit ensuite une étape de séparation, de préférence mécanique, pour former la base de pomme de terre et de végétaux liquide selon l'invention. Cette étape de séparation permet avantageusement de séparer les composés insolubles de l'hydrolysat et la phase liquide étant la base, l'extrait, selon l'invention.

Ainsi, le procédé selon l'invention permet de manière particulièrement avantageuse de préparer une base, un extrait, de pomme de terre et de graines, dont le degré Brix et l'indice de Dextrose Equivalent permettent de qualifier la base d' « extrait », de « sirop » ou de « concentré » ou encore de « poudre d'extrait », « poudre » ou « sirop déshydraté », notamment destinée à la préparation de boissons par dilution, et de fournir un produit facilement utilisable comme produit principal ou dans la préparation de produits intermédiaires ou finaux, ayant un goût, une viscosité, une structure, et un pouvoir sucrant acceptables et recherchés.

L'étape de séchage de l'hydrolysat non séparé, pour former la base de pomme de terre et de végétaux sous forme de poudre, permet ainsi de former une farine hydrolysée de pomme de terre et de végétaux amylacés selon l'invention.

Avantageusement, le deuxième mélange du procédé selon l'invention comprend une teneur en pomme de terre comprise entre 5 et 90% en poids, de préférence comprise entre 10 et 90% en poids, avantageusement comprise entre 15 et 85% en poids, préférentiellement comprise entre 20 et 80% en poids, encore plus avantageusement comprise entre 25 et 75% en poids, encore plus préférentiellement comprise entre 30 et 70% en poids, de préférence comprise entre 35 et 65% en poids, avantageusement comprise entre 40 et 60% en poids.

Préférentiellement, le deuxième mélange du procédé selon l'invention comprend une teneur en végétaux amylacés comprise entre 10 et 95% en poids, de préférence comprise entre 90 et 10% en poids, avantageusement comprise entre 85 et 15% en poids, préférentiellement comprise entre 80 et 20% en poids, encore plus avantageusement comprise entre 75 et 25% en poids, encore plus préférentiellement comprise entre 70 et 30% en poids, de préférence comprise entre 65 et 35% en poids, avantageusement comprise entre 60 et 40% en poids.

Encore plus avantageusement, le deuxième mélange du procédé selon l'invention, comprend une teneur en pomme de terre sous forme de flocons de pomme de terre comprise entre 1 et 92% en poids, de préférence comprise entre 5 et 90%, avantageusement entre 10 et 80%, préférentiellement comprise entre 20 et 70%.

Encore plus préférentiellement, le deuxième mélange du procédé selon l'invention, comprend une teneur en végétaux amylacés comprise entre 8 et 99% en poids, de préférence comprise entre 10 et 95%, avantageusement comprise entre 20 et 90%, préférentiellement comprise entre 30 et 80%.

De préférence, la première étape d'hydrolyse du procédé selon l'invention est réalisée par un chauffage jusqu'à une température apte à solubiliser l'amidon.

Avantageusement, le procédé selon l'invention comprend en outre une étape de refroidissement de l'hydrolysat.

De manière avantageuse, le procédé selon l'invention comprend au moins une deuxième étape d'ajout d'au moins une enzyme, dans la cuve à hydrolyse, après ladite première étape d'hydrolyse, et une deuxième étape d'hydrolyse pour former un deuxième hydrolysat de pomme de terre et de végétaux amylacés.

L'ajout d'au moins une enzyme lors d'une deuxième étape d'ajout dans la cuve à hydrolyse, permet de manière avantageuse d'hydrolyser plus fortement le deuxième mélange de matière première dans la phase aqueuse enzymatique afin d'obtenir un hydrolysat selon la présente invention avec une valeur de Dextrose Equivalent (DE) plus haute, une valeur plus importante de sucres courts et donc in fine un hydrolysat avec un plus haut pouvoir sucrant.

De préférence, le procédé selon l'invention comprend une deuxième étape de chauffe après la deuxième étape d'hydrolyse, jusqu'à désactivation de l'au moins une enzyme ajoutée lors de l'au moins une deuxième étape d'ajout

Avantageusement, l'au moins une enzyme du procédé selon l'invention est choisie dans le groupe comprenant les hydrolases (3. -. -.-).

Plus particulièrement, l'au moins une enzyme du procédé selon l'invention est choisie dans le groupe comprenant les estérases (3. 1. -.-), les glycosylases (3.2. -.-), les éther-hydrolases (3.3. -.-), les peptidases (3. 4. -.-), les enzymes de la classe 3. 5. -.-, les enzymes de la classe 3. 6. -. , les enzymes de la classe 3. 7. -.-, les enzymes de la classe 3. 8. -.-, les enzymes de la classe 3. 9. -.-, les enzymes de la classe 3. 10. -.-, les enzymes de la classe 3. 11. -.-, les enzymes de la classe 3. 12. -.-, les enzymes de la classe 3. 13. -.-, les enzymes de la classe 5. 3.

De manière avantageuse, le procédé selon l'invention comprend en outre une étape de concentration de la base de pomme de terre et de végétaux sous forme liquide, de préférence dans un évaporateur, pour former une base de pomme de terre et de végétaux liquide concentrée.

De manière particulièrement avantageuse, le procédé selon l'invention comprend en outre une étape de séchage de la base de pomme de terre et de végétaux sous forme liquide, pour former une base de pomme de terre et de végétaux solide, de préférence sous forme de poudre.

D'autres formes de réalisation du procédé de préparation d'une base de pomme de terre et de végétaux amylacés selon la présente invention sont indiquées dans les revendications annexées.

L'invention a aussi pour objet une utilisation d'une base de pomme de terre et de végétaux amylacés selon la présente invention et/ou obtenue par le procédé selon l'invention comme produit principal ou comme produit intermédiaire destiné à être utilisé dans l'industrie agro-alimentaire et/ou cosmétique et/ou pharmaceutique.

De préférence, la base est utilisée pour la préparation de base pour produits végétaux et/ou de base pour produits laitier et/ou de produit alimentaire choisi dans le groupe comprenant les boissons (notamment, soda, laitière, végétale), les desserts et yaourts (laitiers et végétaux), les biscuits, barres céréalières, en-cas, les céréales pour petit-déjeuner, les biscottes, crackers, les confiseries, les pains, pâtisseries, cakes, gaufres, les crèmes glacées, les sauces, les aliments pour bébé, les produits diététiques/sportifs, les préparations fruitées (notamment, confitures et compotes), et/ou dans la préparation d'un produit cosmétique et/ou pharmaceutique.

Il est bien entendu que la base de pomme de terre et de végétaux amylacés selon l'invention est une base de pomme de terre et d'un ou plusieurs végétaux amylacés, par exemple deux végétaux amylacés ou plus, trois végétaux amylacés ou plus, quatre végétaux amylacés ou plus.

D'autres formes de réalisation de l'utilisation d'une base de pomme de terre et de végétaux amylacés selon la présente invention sont indiquées dans les revendications annexées.

### Description détaillée d'une réalisation de l'invention

D'autres caractéristiques et avantages de la présente invention seront tirés de la description non limitative qui suit, et en faisant référence et aux exemples.

Une série de bases de pomme de terre et de végétaux amylacés selon la présente invention ont été préparés.

En effet, la série de bases de pomme de terre et de végétaux amylacés selon l'invention, se présentent sous forme liquide ou sous forme solide, par exemple de poudre, et présentent sous forme liquide un degré Brix compris entre 65 et 85, une viscosité mesurée à 25°C comprise entre 500 et 39 000 mPa.s, sous forme de poudre une teneur en matière sèche comprise entre 90% et 100%, et une valeur de Dextrose Equivalent (DE) comprise entre 10 et 95.

Il est particulièrement avantageux que de telles bases de pomme de terre et de végétaux selon l'invention, puissent être utilisé pour leur pouvoir sucrant, dans un produit intermédiaire ou final qui soit alimentaire, cosmétique et/ou pharmaceutique, en fournissant un bon compromis entre la teneur en sucres, la viscosité, et la durée de conservation, en fonction de la base selon l'invention utilisée et du résultat recherché dans le produit ultérieur.

En outre, en fonction de la valeur de Dextrose Equivalent (DE), les bases de pomme de terre et de végétaux amylacés selon l'invention présentent plusieurs avantages tels qu'un goût particulier, une couleur particulière, une viscosité ou une texture particulièrement recherchés ainsi qu'étant utilisés comme agent de charge.

On a préparé une série de 6 bases de pomme de terre et de maïs selon l'invention, sous forme liquide concentrée, sous forme liquide de type jus non concentré, et étant éventuellement ou préférablement séchées sous forme de poudre.

La première base de pomme de terre et de maïs selon l'invention, sous forme liquide concentrée, en hydrolysant un mélange de 52,6% de pomme de terre et de 47,4% de maïs ou en hydrolysant un mélange de 20,5% de flocons de pomme de terre et 79,5% de maïs. Une telle première base liquide concentrée présentant une valeur visée de Dextrose Equivalent de 10, une teneur en sucres de 3,5% sur matière sèche, une valeur Brix de 55 et une viscosité de 2500 mPa.s sous forme liquide. Cette première base liquide concentrée est préférablement séchée pour obtenir une poudre de pomme de terre et de maïs.

La deuxième base de pomme de terre et de maïs selon l'invention, sous forme de sirop liquide concentrée, en hydrolysant un mélange de 52,6% de pomme de terre et de 47,4% de maïs ou en hydrolysant un mélange de 20,5% de flocons de pomme de terre et 79,5% de maïs. Une telle deuxième base présentant une valeur visée de Dextrose Equivalent de 25, une teneur en sucres de 8,5% sur matière sèche, une valeur Brix de 71 et une viscosité de 39000 mPa.s sous forme liquide. Cette deuxième base liquide concentrée est éventuellement séchée pour obtenir une poudre de pomme de terre et de maïs.

La troisième base de pomme de terre et de maïs selon l'invention, sous forme de sirop liquide concentrée, en hydrolysant un mélange de 52,6% de pomme de terre et de 47,4% de maïs ou en hydrolysant un mélange de 20,5% de flocons de pomme de terre et 79,5% de maïs. Une telle troisième base présentant une valeur visée de Dextrose Equivalent de 45, une teneur en sucres de 42% sur matière sèche, une valeur Brix de 77 et une viscosité de 5000 mPa.s sous forme liquide.

La quatrième base de pomme de terre et de maïs selon l'invention, sous forme liquide de type jus non concentré, en hydrolysant un mélange de 52,6% de pomme de terre et de 47,4% de maïs ou en hydrolysant un mélange de 20,5% de flocons de pomme de terre et 79,5% de maïs. Une telle quatrième base présentant une valeur visée de Dextrose Equivalent de 45, une teneur en sucres de 42% sur matière sèche, une valeur Brix de 24 et une viscosité comprise entre 1 et 20 mPa.s.

La cinquième base de pomme de terre et de maïs selon l'invention, sous forme liquide concentrée, en hydrolysant un mélange de 52,6% de pomme de terre et de 47,4% de maïs ou en hydrolysant un mélange de 20,5% de flocons de pomme de terre et 79,5% de maïs. Une telle cinquième base présentant une valeur de Dextrose Equivalent de 62, une teneur en sucres de 69% sur matière sèche, une valeur Brix de 80 et une viscosité de 8500 mPa.s sous forme liquide.

La sixième base de pomme de terre et de maïs selon l'invention, sous forme de sirop liquide ou de poudre, en hydrolysant un mélange de 52,6% de pomme de terre et de 47,4% de maïs ou en hydrolysant un mélange de 20,5% de flocons de pomme de terre et 79,5% de maïs. Une telle sixième base présentant une valeur visée de Dextrose Equivalent de 95, une teneur en sucres de 83,5% sur matière sèche, une valeur Brix de 72 et une viscosité de 500 mPa.s sous forme liquide. Cette sixième base liquide concentrée est éventuellement séchée pour obtenir une poudre de pomme de terre et de maïs.

On a ensuite préparé une base de pomme de terre et d'épeautre selon l'invention, sous forme de sirop liquide concentrée, en hydrolysant un mélange de 52,6% de pomme de terre et de 47,4% d'épeautre ou en hydrolysant un mélange de 20,5% de flocons de pomme de terre et 79,5% d'épeautre. Une telle base présentant une valeur visée de Dextrose Equivalent de 45, une teneur en sucres de 51,5% sur matière sèche, une valeur Brix de 76 et une viscosité de 5000 mPa.s sous forme liquide.

On a également préparé une base de pomme de terre et de manioc selon l'invention, sous forme de sirop liquide concentrée, en hydrolysant un mélange de 52,6% de pomme de terre et de 47,4% de manioc ou en hydrolysant un mélange de 20,5% de flocons de pomme de terre et 79,5% de manioc. Une telle base présentant une valeur visée de Dextrose Equivalent de 35, une teneur en sucres de 38,5% sur matière sèche, une valeur Brix de 79 et une viscosité de 14500 mPa.s sous forme liquide. Cette base liquide concentrée de pomme de terre/manioc est éventuellement séchée pour obtenir une poudre de pomme de terre/manioc.

On a aussi préparé une base de pomme de terre et de manioc selon l'invention, sous forme liquide de type jus non concentré, en hydrolysant un mélange de 52,6% de pomme de terre et de 47,4% de manioc ou en hydrolysant un mélange de 20,5% de flocons de pomme de terre et 79,5% de manioc. Une telle base présentant une valeur de Dextrose Equivalent de 35, une teneur en sucres de 38,5% sur matière sèche, une valeur Brix de 30 et une viscosité comprise entre 1 et 20 mPa.s sous forme liquide.

En outre, on a préparé deux bases de pomme de terre et d'avoine selon l'invention.

La première base de pomme de terre et d'avoine selon l'invention, sous forme de sirop liquide concentrée, en hydrolysant un mélange de 52,6% de pomme de terre et de 47,4% d'avoine. Une telle base présentant une valeur visée de Dextrose Equivalent de 35, une teneur en sucres de 36,5% sur matière sèche, une valeur Brix de 78 et une viscosité de 8000 mPa.s sous forme liquide. Cette base liquide concentrée de pomme de terre/avoine est éventuellement séchée pour obtenir une poudre de pomme de terre/avoine.

La deuxième base de pomme de terre et d'avoine selon l'invention, sous forme de sirop liquide concentrée, en hydrolysant un mélange de 52,6% de pomme de terre et de 47,4% d'avoine. Une telle base présentant une valeur visée de Dextrose Equivalent de 52, une teneur en sucres de 62,5% sur matière sèche, une valeur Brix de 80 et une viscosité de 21000 mPa.s sous forme liquide.

En outre, on a également préparé une base de pomme de terre et de millet selon l'invention, sous forme de sirop liquide concentrée, en hydrolysant un mélange de 52,6% de pomme de terre et de 47,4% de millet ou en hydrolysant un mélange de 20,5% de flocons de pomme de terre et 79,5% de millet. Une telle base présentant une valeur visée de Dextrose Equivalent de 45, une teneur en sucres de 43% sur matière sèche, une valeur Brix de 76 et une viscosité de 8000 mPa.s sous forme liquide.

On a enfin préparé une nouvelle série de bases de pomme de terre et de riz selon l'invention.

La première base de pomme de terre et de riz selon l'invention, sous forme de sirop liquide concentrée, en hydrolysant un mélange de 90% de pomme de terre et de 10% de riz ou en hydrolysant un mélange de 67,6% de flocons de pomme de terre et 32,4% de riz. Une telle base présentant une valeur visée de Dextrose Equivalent de 45, une teneur en sucres de 39% sur matière sèche, une valeur Brix de 76 et une viscosité de 5500 mPa.s sous forme liquide.

La deuxième base de pomme de terre et de riz selon l'invention, sous forme liquide de type jus non concentré, en hydrolysant un mélange de 90% de pomme de terre et de 10% de riz ou en hydrolysant un mélange de 67,6% de flocons de pomme de terre et 32,4% de riz. Une telle base présentant une valeur visée de Dextrose Equivalent de 45, une teneur en sucres de 39% sur matière sèche, une valeur Brix de 17 et viscosité comprise entre 1 et 20 mPa.s sous forme liquide.

La troisième base de pomme de terre et de riz selon l'invention, sous forme de sirop liquide concentrée, en hydrolysant un mélange de 70% de pomme de terre et de 30% de riz ou en hydrolysant un mélange de 35,2% de flocons de pomme de terre et 64,8% de riz. Une telle base présentant une valeur visée de Dextrose Equivalent de 45, une teneur en sucres de 36% sur matière sèche, une valeur Brix de 77 et une viscosité de 6500 mPa.s sous forme liquide.

La quatrième base de pomme de terre et de riz selon l'invention, sous forme de sirop liquide concentrée, en hydrolysant un mélange de 52,6% de pomme de terre et de 47,4% de riz ou en hydrolysant un mélange de 20,5% de flocons de pomme de terre et 79,5% de riz. Une telle base présentant une valeur visée de Dextrose Equivalent de 45, une teneur en sucres de 42,5% sur matière sèche, une valeur Brix de 75 et une viscosité de 2000 mPa.s sous forme liquide.

On a également préparé une base de pomme de terre/maïs/manioc ainsi qu'une base de pomme de terre/maïs/millet.

La base de pomme de terre/maïs/manioc selon l'invention, sous forme de sirop liquide concentrée, en hydrolysant un mélange de 34.0% de pomme de terre, de 33% de maïs et de 33% de manioc ou en hydrolysant un mélange de 10.7% de flocons de pomme de terre, de 44.4% de maïs et de 44.6% de manioc. Une telle base présentant une valeur visée de Dextrose Equivalent de 35, une teneur en sucres de 37% sur matière sèche, une valeur Brix de 76 et une viscosité de 4500 mPa.s sous forme liquide.

La base de pomme de terre/maïs/millet selon l'invention, sous forme de sirop liquide concentrée, en hydrolysant un mélange de 34.0 % de pomme de terre, de 33% de maïs et de 33% de millet ou en hydrolysant un mélange de 10.7% de flocons de pomme de terre, de 44.6% de maïs et de 44.6% de millet. Une telle base présentant une valeur visée de Dextrose Equivalent de 40, une teneur en sucres de 35% sur matière sèche, une valeur Brix de 76 et une viscosité de 4000 mPa.s sous forme liquide.

Ainsi, la série de bases de pomme de terre et de maïs, d'épeautre, de manioc, d'avoine, de millet ou encore de riz ou même de plusieurs végétaux amylacés tels qu'un mélange de maïs et manioc ou bien de maïs et millet, préparées selon l'invention sous forme liquide de type jus non concentré, sous forme liquide concentré ou encore sous forme de poudre, permettent de valoriser les filières agricoles locales, permettent d'éviter que ces denrées ne soient jetées ou détruites sans valorisation et peuvent être utilisées directement ou dans la préparation de produits intermédiaires ou finaux selon le goût, pouvoir sucrant, couleur, viscosité et/ou structure recherchés.

De manière avantageuse, l'étape d'hydrolyse a eu lieu de manière concomitante pour l'hydrolyse respectivement de la pomme de terre avec le maïs, l'épeautre, le manioc, l'avoine, le millet ou encore le riz ou même les mélanges maïs/manioc et maïs/millet afin d'obtenir les bases selon l'invention décrites ci-avant, avec des valeurs de Brix, de Dextrose Equivalent, de viscosité et de teneurs en sucres, contrôlées et sélectionnées en fonction des utilisations ultérieures de telles bases.

Par exemple pour la préparation de base pour produits végétaux et/ou de base pour produits laitier et/ou de produit alimentaire choisi dans le groupe comprenant les boissons (notamment, soda, laitière, végétale), les desserts et yaourts (laitiers et végétaux), les biscuits, barres céréalières, en-cas, les céréales pour petit-déjeuner, les biscottes, crackers, les confiseries, les pains, pâtisseries, cakes, gaufres, les crèmes glacées, les sauces, les aliments pour bébé, les produits diététiques/sportifs, les préparations fruitées (notamment, confitures et compotes), et/ou dans la préparation d'un produit cosmétique et/ou pharmaceutique.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Base de pomme de terre et de végétaux amylacés, sous forme liquide ou sous forme de poudre, comprenant du glucose, du sucrose et du maltose, présentant un degré Brix compris entre 65 et 85 sous forme liquide, de préférence compris entre 66 et 84, de préférence entre 67 et 83, préférentiellement entre 68 et 82, de manière préférée entre 69 et 81, plus précisément entre 70 et 80, par exemple entre 71 et 80, ou une teneur en matière sèche comprise entre 90% et 100% sous forme de poudre, de préférence comprise entre 92% et 100%, de préférence comprise entre 92% et 98%, préférentiellement comprise entre 94% et 98%, et présentant une valeur de Dextrose Equivalent (DE) comprise entre 10 et 95, de préférence comprise entre 15 et 90, de préférence entre 15 et 85, préférentiellement entre 15 et 89, de manière avantageusement entre 20 et 75, de préférence comprise entre 25 et 70, avantageusement comprise entre 30 et 65, plus précisément comprise entre 35 et 65, de manière avantageuse comprise entre 35 et 62.

2. Base de pomme de terre et de végétaux amylacés selon la revendication 1, sous forme liquide présentant une viscosité mesurée à 25°C comprise entre 1 et 50000 mPa.s, de préférence comprise entre 500 et 39000 mPa.s, avantageusement comprise entre 1000 et 35000 mPa.s, préférentiellement comprise entre 2000 et 30000 mPa.s, plus préférentiellement comprise entre 3000 et 28000 mPa.s, encore plus avantageusement comprise entre 4000 et 26000 mPa.s, avantageusement comprise entre 5000 et 25000 mPa.s, de manière préférée comprise entre 5000 et 21000 mPa.s.

3. Base de pomme de terre et de végétaux amylacés selon l'une quelconque des revendications 1 ou 2, présentant un pH compris entre 3,5 et 7,5.

4. Base de pomme de terre et de végétaux amylacés selon l'une quelconque des revendications 1 à 3, comprenant une quantité en sucres sur matière sèche comprise entre 2% et 86% en poids, de préférence comprise entre 3,5% et 85% en poids, préférentiellement comprise entre 5% et 80%, avantageusement comprise entre 10% et 80%, de manière avantageuse comprise entre 15% et 80%, de manière préférée comprise entre 20% et 80%, plus préférentiellement comprise entre 25% et 80%, avantageusement comprise entre 25% et 75%, de préférence comprise entre 30% et 75%, de manière préférée comprise entre 35% et 75%, de manière encore plus préférée comprise entre 35% et 70%.

5. Base de pomme de terre et de végétaux amylacés selon l'une quelconque des revendications 1 à 4, présentant une teneur en potassium comprise entre 100 et 4500 ppm et/ou présentant une teneur en magnésium comprise entre 30 et 1000 ppm et/ou présentant une teneur en phosphore comprise entre 40 et 3500 ppm et/ou présentant une teneur en calcium comprise entre 20 et 200 ppm et/ou présentant une teneur en fer comprise entre 1 et 6 ppm et/ou présentant une teneur en gluten inférieure à 100 ppm, de préférence inférieure à 80 ppm, préférentiellement inférieure à 60 ppm, avantageusement inférieure à 50 ppm, de manière particulièrement avantageuse inférieure à 40 ppm, de manière encore plus préférée inférieure à 30 ppm, idéalement inférieure à 20 ppm, plus précisément inférieure à 10 ppm, voire inférieure à 5 ppm.

6. Base de pomme de terre et de végétaux amylacés selon l'une quelconque des revendications 1 à 5, dans laquelle lesdites végétaux amylacés sont des céréales, des pseudo-céréales, des poaceaes, des amaranthaceaes, des polygonaceaes, des fabaceaes, les plantes racines, les convolvulaceaes, les euphorbiaceaes, les apiaceaes, les asteraceaes et leurs mélanges, de préférence lesdits végétaux amylacés sont choisies dans le groupe comprenant le riz, l'avoine, le blé, le manioc, l'épeautre, le seigle, le maïs, l'orge malté, le teff, le sorgho, le quinoa, le sarrasin, le millet, l'orge, les pois, les pois chiche, l'amaranthe, le blé dur, le tritordeum, le tournesol, et leurs mélanges.

7. Procédé de préparation d'une base de pomme de terre et de végétaux amylacés selon l'une des revendications 1 à 6, comprenant :
- une alimentation d'une unité de préparation en végétaux amylacés,
- une alimentation d'une cuve à hydrolyse avec de la pomme de terre, et une phase aqueuse,
- au moins une première étape d'ajout d'au moins une enzyme dans la cuve à hydrolyse pour former un premier mélange de pomme de terre dans une phase aqueuse enzymatique,
- un entraînement progressif et continu pendant une période de temps prédéterminé, dans un circuit de circulation, des végétaux amylacés de l'unité de préparation par ledit premier mélange, pour former progressivement et en continu un deuxième mélange de végétaux dans la phase aqueuse enzymatique comprenant la pomme de terre, de préférence ledit deuxième mélange comprend une teneur en pomme de terre comprise entre 5 et 90% en poids, de préférence comprise entre 10 et 90% en poids, avantageusement comprise entre 15 et 85% en poids, préférentiellement comprise entre 20 et 80% en poids, encore plus avantageusement comprise entre 25 et 75% en poids, encore plus préférentiellement comprise entre 30 et 70% en poids, de préférence comprise entre 35 et 65% en poids, avantageusement comprise entre 40 et 60% en poids, et/ou ledit deuxième mélange comprend une teneur en végétaux amylacés comprise entre 95 et 10% en poids, de préférence comprise entre 90 et 10% en poids, avantageusement comprise entre 85 et 15% en poids, préférentiellement comprise entre 80 et 20% en poids, encore plus avantageusement comprise entre 75 et 25% en poids, encore plus préférentiellement comprise entre 70 et 30% en poids, de préférence comprise entre 65 et 35% en poids, avantageusement comprise entre 60 et 40% en poids,
- une première étape d'hydrolyse progressive et continue du deuxième mélange pour former un hydrolysat de pomme de terre et de végétaux amylacés,
- une étape de séparation mécanique de l'hydrolysat pour former la base de pomme de terre et de végétaux sous forme liquide, ou
- une étape de séchage de l'hydrolysat pour former la base de pomme de terre et de végétaux sous forme de poudre.

8. Procédé de préparation d'une base de pomme de terre et de végétaux amylacés selon l'une quelconque des revendications 1 à 6, comprenant :
- une alimentation d'une cuve à hydrolyse avec une phase aqueuse,
- une série d'alimentations d'une unité de préparation en végétaux amylacés et en flocons ou farine de flocons de pomme de terre,
- au moins une première étape d'ajout d'au moins une enzyme dans la cuve à hydrolyse,
- un entraînement progressif et continu pendant une période de temps prédéterminé, dans un circuit de circulation, des végétaux amylacés et des flocons ou farine de flocons de pomme de terre de l'unité de préparation par la phase aqueuse enzymatique, de préférence le deuxième mélange comprend une teneur en pomme de terre sous forme de flocons de pomme de terre comprise entre 1 et 92% en poids, de préférence comprise entre 5 et 90%, avantageusement entre 10 et 80%, préférentiellement comprise entre 20 et 70%, et/ou le deuxième mélange comprend une teneur en végétaux amylacés comprise entre 8 et 99% en poids, de préférence comprise entre 10 et 95%, avantageusement comprise entre 20 et 90%, préférentiellement comprise entre 30 et 80%,
- une étape d'hydrolyse progressive et en continue du mélange de végétaux amylacés dans la phase aqueuse enzymatique comprenant les flocons ou farine de flocons de pomme de terre,
- une étape de séparation mécanique de l'hydrolysat pour former la base de pomme de terre et de végétaux sous forme liquide, ou
- une étape de séchage de l'hydrolysat pour former la base de pomme de terre et de végétaux sous forme de poudre.

9. Procédé selon l'une quelconque des revendications 7 ou 8, dans lequel ladite première étape d'hydrolyse est réalisée par un chauffage jusqu'à une température apte à solubiliser l'amidon.

10. Procédé selon la revendication 9, comprenant en outre une étape de refroidissement de l'hydrolysat.

11. Procédé selon l'une des revendications 7 à 10, comprenant au moins une deuxième étape d'ajout d'au moins une enzyme, dans la cuve à hydrolyse, après ladite première étape d'hydrolyse, et une deuxième étape d'hydrolyse pour former un deuxième hydrolysat de pomme de terre et de végétaux amylacés, de préférence comprenant une deuxième étape de chauffe après ladite deuxième étape d'hydrolyse, jusqu'à désactivation de ladite au moins une enzyme ajoutée lors de l'au moins une deuxième étape d'ajout.

12. Procédé selon l'une quelconque des revendications 7 à 11, dans lequel ladite au moins une enzyme est choisie dans le groupe comprenant les hydrolases (3. -. -.-), de préférence ladite au moins une enzyme est choisie dans le groupe comprenant les estérases (3. 1. -.-), les glycosylases (3.2. -.-), les éther-hydrolases (3.3. -.-), les peptidases (3. 4. -. ), les enzymes de la classe 3. 5. -.-, les enzymes de la classe 3. 6. -.-, les enzymes de la classe 3. 7. -.-, les enzymes de la classe 3. 8. -.-, les enzymes de la classe 3. 9. -.-, les enzymes de la classe 3. 10. -.-, les enzymes de la classe 3. 11. -.-, les enzymes de la classe 3. 12. -.-, les enzymes de la classe 3. 13. -.-, les enzymes de la classe 5. 3.

13. Procédé selon l'une quelconque des revendications 7 à 12, comprenant en outre une étape de concentration de la base de pomme de terre et de végétaux amylacés sous forme liquide, de préférence dans un évaporateur, pour former une base de pomme de terre et de végétaux amylacés liquide concentrée.

14. Procédé selon l'une quelconque des revendications 7 à 13, comprenant en outre une étape de séchage de la base de pomme de terre et de végétaux amylacés sous forme liquide, pour former une base de pomme de terre et de végétaux amylacés solide, de préférence sous forme de poudre.

15. Utilisation de la base de pomme de terre et de végétaux amylacés selon l'une quelconque des revendications 1 à 6 et/ou obtenue par le procédé selon l'une quelconque des revendications 7 à 14 comme produit principal ou comme produit intermédiaire destiné à être utilisé dans l'industrie agro-alimentaire et/ou cosmétique et/ou pharmaceutique, de préférence ladite base est utilisée pour la préparation de base pour produits végétaux et/ou de base pour produits laitier et/ou de produit alimentaire choisi dans le groupe comprenant les boissons (notamment, soda, laitière, végétale), les desserts et yaourts (laitiers et végétaux), les biscuits, barres céréalières, en-cas, les céréales pour petit-déjeuner, les biscottes, crackers, les confiseries, les pains, pâtisseries, cakes, gaufres, les crèmes glacées, les sauces, les aliments pour bébé, les produits diététiques/sportifs, les préparations fruitées (notamment, confitures et compotes), et/ou dans la préparation d'un produit cosmétique et/ou pharmaceutique.
